# EUROPÄISCHE PATENTANMELDUNG

(11) **EP 2 184 009 A2**
(43) Veröffentlichungstag der Anmeldung: **12.05.2010**
(21) Anmeldenummer: 09172867.5
(22) Anmeldetag: 13.10.2009
(51) Int. Cl.: A61B 5/0464

(54) **Einkammer-Herzstimulator**

(30) Priorität: 05.11.2008 DE 102008043480
(71) Anmelder: Biotronik CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Die Erfindung betrifft einen Einkammer-Herzstimulator gelöst, der ein wenigstens zum Teil elektrisch leitendes Gehäuse aufweist, sowie eine ventrikuläre Sensingeinheit, eine weitere Erfassungseinheit und eine VT/SVT-Diskriminierungseinheit. Die ventrikuläre Sensingeinheit ist als erste Erfassungseinheit über eine ventrikuläre Elektrodenleitung mit wenigstens einem ventrikulären Sensingelektrodenpaar verbunden und ist ausgebildet, ventrikuläre Herzaktionen vermittels eines über ein ventrikuläres Sensingelektrodenpaar bipolar aufgenommenen ersten Elektrokardiogrammsignals wahrzunehmen und zu klassifizieren. Die weitere Erfassungseinheit ist einerseits über eine ventrikuläre Elektrodenleitung mit wenigstens einer Elektrode dieser ventrikulären Elektrodenleitung verbunden und andererseits mit dem elektrisch leitenden Gehäuse des Einkammer-Herzstimulators. Die weitere Erfassungseinheit ist ausgebildet, in einem über diese Elektroden aufgenommenen zweiten Elektrokardiogrammsignal für atriale Herzaktionen charakteristische Signalmerkmale zu detektieren. Die VT/SVT-Diskriminierungseinheit ist mit der ventrikulären Sensingeinheit und der weiteren Erfassungseinheit verbunden und ausgebildet, anhand über die ventrikuläre Sensingeinheit erfasster ventrikulärer Herzaktionen sowie von über die weitere Erfassungseinheit erfassten atrialen Herzaktionen eine Unterscheidung zwischen ventrikulären und superventrikulären Tachykardien durchzuführen, sofern die Rate der erfassten ventrikulären Herzaktionen einen Grenzwert (VT-Zonen-Grenzwert) überschreitet.

## Beschreibung

Die Erfindung betrifft einen Einkammer-Herzstimulator, insbesondere einen implantierbaren Herzschrittmacher oder einen implantierbaren Cardioverter/Defibrillator, mit einer rechtsventrikulären Elektrodenleitung. An dieser Elektrodenleitung sind wenigstens eine rechtsventrikuläre Sensingelektrode und eine rechtsventrikuläre Stimulationselektrode angebracht, die im Einzelfall auch von einem einzigen Elektrodenpol gebildet sein können, der dann sowohl als Stimulations- als auch als Sensingelektrode dient.

Unter einem Einkammer-Herzstimulator wird hier ein Herzstimulator verstanden, der nach üblicher Nomenklatur dazu in der Lage ist, nur in einer Herzkammer ein intrakardiales Elektrokardiogramm über eine entsprechende Sensingelektrode aufzunehmen und nur an diese Herzkammer Stimulationsimpulse über eine entsprechende Stimulationselektrode abzugeben. Stimulations- und Sensingelektrode können dabei verschieden voneinander sein oder von demselben Elektrodenpol gebildet sein. Unter einem Einkammer-Herzstimulator soll hier auch ein Herzstimulator verstanden werden, der grundsätzlich auch mit Sensingelektroden in mehreren Herzkammern verbunden werden und so als Mehrkammer-Herzstimulator dienen kann, der aber im Betrieb nur mit einer Elektrodenleitung zum Sensing und zur Stimulation in einer Herzkammer verbunden ist.

Ein Vorteil eines derartigen Einkammer-Herzstimulators ist dessen grundsätzlich einfacher Aufbau gepaart mit der Tatsache, dass im Herzen eines Patienten nur eine einzige Elektrodenleitung implantiert zu werden braucht.

Derzeit gibt es verschiedene im Markt anerkannte Algorithmen zur Diskriminierung zwischen behandlungspflichtigen ventrikulären Tachykardien (VT) und supraventrikulären Tachykardien (SVT), die keiner Therapie bedürfen. Derartige Algorithmen sind in den implantierbaren Kardioverter/Defibrillatoren (ICD) aller Hersteller verfügbar und deren Wirksamkeit (Sensitivität/Spezifität) wurde in klinischen Studien nachgewiesen. Allerdings sind die derzeit bekannten Algorithmen nur in sog. Zwei- oder Dreikammer-IDCs verfügbar, da diese immer auch die Informationen des Vorhofs benötigen.

Für Einkammer-ICDs, d.h. solche ohne eine implantierte Elektrode im Vorhof gibt es diese Diskriminierungsalgorithmen nicht, jedoch stehen hier verschiedene Zusatzkriterien zur Detektionsverbesserung zur Verfügung (Sudden Onset, Stabilität, verschiedene QRS-Morphologiekriterien). Diese Kriterien sind jedoch hinsichtlich der zu erwartenden Sensitivität/Spezifität beschränkt und können nicht alle Formen der VTs von SVTs unterscheiden. Insbesondere ist der Nachweis der Spezifitätsverbesserung bei Einsatz von Morphologiekriterien in klinischen Studien in der Vergangenheit mehrfach gescheitert.

Aufgabe der Erfindung ist es, einen Einkammer-Herzstimulator zu schaffen, der eine verbesserte VT/SVT-Diskriminierung ermöglicht.

Erfindungsgemäß wird diese Aufgabe durch einen Einkammer-Herzstimulator gelöst, der ein wenigstens zum Teil elektrisch leitendes Gehäuse aufweist, sowie eine ventrikuläre Sensingeinheit, eine weitere Erfassungseinheit und eine VT/SVT-Diskriminierungseinheit. Die ventrikuläre Sensingeinheit ist als erste Erfassungseinheit über eine ventrikuläre Elektrodenleitung mit wenigstens einem ventrikulären Sensingelektrodenpaar verbunden oder zu verbinden und ist ausgebildet, ventrikuläre Herzaktionen vermittels eines über ein ventrikuläres Sensingelektrodenpaar bipolar aufgenommenen ersten Elektrokardiogrammsignals wahrzunehmen und zu klassifizieren, d.h. ventrikuläre Herzaktionen zu detektieren. Dies geschieht beispielsweise dadurch, dass in dem aufgenommenen Elektrokardiogrammsignal QRS-Komplexe durch Schwellwertvergleich detektiert werden. Die weitere Erfassungseinheit ist einerseits über eine ventrikuläre Elektrodenleitung mit wenigstens einer Elektrode dieser ventrikulären Elektrodenleitung verbunden oder zu verbinden und andererseits mit dem elektrisch leitenden Gehäuse des Einkammer-Herzstimulators. Die weitere Erfassungseinheit ist ausgebildet, in einem über diese Elektroden aufgenommenen zweiten Elektrokardiogrammsignal für atriale Herzaktionen charakteristische Signalmerkmale zu detektieren. Solche für atriale Herzaktionen charakteristische Signalmerkmale sind als P-Wellen bekannt. Die VT/SVT-Diskriminierungseinheit ist mit der ventrikulären Sensingeinheit und der weiteren Erfassungseinheit verbunden und ausgebildet, anhand über die ventrikuläre Sensingeinheit erfasster ventrikulärer Herzaktionen sowie von über die weitere Erfassungseinheit erfassten atrialen Herzaktionen eine Unterscheidung zwischen ventrikulären und superventrikulären Tachykardien durchzuführen, sofern die Rate der erfassten ventrikulären Herzaktionen einen Grenzwert (VT-Zonen-Grenzwert) überschreitet.

Vorzugsweise ist die weitere Erfassungseinheit ausgebildet, für atriale Herzaktionen charakteristische Signalmerkmale, also P-Wellen, vor und während einer Tachykardie zu detektieren.

Außerdem ist es bevorzugt, dass die weitere Erfassungseinheit ausgebildet ist, für atriale Herzaktionen charakteristische Signalmerkmale durch Signalermittlung über mehrere Herzzyklen zu bestimmen.

Zur Aufnahme des zweiten Elektrokardiogramms ist die weitere Erfassungseinheit vorzugsweise mit einer Schockwendel an einer ventrikulären Elektrodenleitung sowie mit dem elektrisch leitenden Gehäuse des Herzstimulators verbunden. Wenn die ventrikuläre Elektrodenleitung zwei solcher Schockwendeln aufweist, beispielsweise eine distale, zur Platzierung im Ventrikel vorgesehene Schockwendel und eine proximale, zur Platzierung in der Vena cava superior vorgesehene Schockwendel, kann die weitere Erfassungseinheit wahlweise mit einer dieser beiden Schockwendeln und dem elektrisch leitenden Gehäuse des Herzstimulators verbunden sein. Alternativ kann die weitere Erfassungseinheit auch mit einer ventrikulären Spitzenelektrode oder einer ventrikulären Ringelektrode an der ventrikulären Elektrodenleitung als einer ersten Elektrode und dem elektrisch leitenden Gehäuse des Herzstimulators als zweiter Elektrode verbunden sein.

In diesem Zusammenhang ist es bevorzugt, dass die weitere Erfassungseinheit über eine Schaltmatrix wahlweise mit wenigstens einer von mehreren Elektroden einer ventrikulären Elektrodenleitung verbunden ist oder zu verbinden ist. Dies erlaubt es, diejenige Elektrode der ventrikulären Elektrodenleitung, mit der die weitere Erfassungseinheit verbunden werden soll, gezielt auszuwählen. Hierzu ist vorzugsweise eine Auswahleinheit vorgesehen, die mit der Schaltmatrix verbunden ist und die ausgebildet ist, die Schaltmatrix basierend auf einer EKG-Signalqualitätsprüfung automatisch so umzuschalten, dass die weitere Erfassungseinheit automatisch mit derjenigen Elektrode der ventrikulären Elektrodenleitung verbunden wird, die das geeignetste Elektrokardiogrammsignal liefert.

In einer weiteren bevorzugten Ausführungsvariante kann die weitere Erfassungseinheit auch dazu ausgebildet sein, mehrere zweite Elektrokardiogrammsignale gleichzeitig aufzunehmen und miteinander zu mitteln.

Ein bevorzugter Einkammer-Herzstimulator weist außerdem eine Zeitmesseinheit auf, die ausgebildet ist, den zeitlichen Abstand aufeinander folgender ventrikulärer Herzaktionen als jeweiliges RR-Intervall oder dessen Kehrwert als jeweilige ventrikuläre Rate zu bestimmen und den so bestimmten Wert der VT/SVT-Diskriminierungseinheit zuzuführen. In diesem Zusammenhang ist die VT/SVT-Diskriminierungseinheit ausgebildet, ein jeweiliges RR-Intervall oder eine jeweilige ventrikuläre Rate mit wenigstens einem VT-Zonen-Grenzwert zu vergleichen und für den Fall, dass ein jeweiliges RR-Intervall kürzer ist als der entsprechende VT-Zonen-Grenzwert oder eine jeweilige ventrikuläre Rate größer ist als der entsprechende VT-Zonen-Grenzwert, eine Tachykardie zu detektieren und in dem Fall, dass ein jeweiliges RR-Intervall länger ist als der entsprechende VT-Zonen-Grenzwert oder eine jeweilige ventrikuläre Rate geringer ist als der entsprechende VT-Zonen-Grenzwert, einen Normalrhythmus zu detektieren.

In einer besonders bevorzugten Ausführungsvariante ist der Herzstimulator zu einer weiteren Diskriminierung des Herzrhythmus dadurch in der Lage, dass die Diskriminierungseinheit ein jeweiliges RR-Intervall oder eine jeweilige ventrikuläre Rate mit wenigstens zwei VT-Zonen-Grenzwerten zu vergleichen und eine Tachykardie zu erkennen, wenn das jeweilige RR-Intervall oder die jeweilige ventrikuläre Rate zwischen den beiden VT-Zonen-Grenzwerten liegt und eine ventrikuläre Fibrillation zu detektieren, wenn das jeweilige RR-Intervall kürzer ist als beide VT-Zonen-Grenzwerte bzw. die ventrikuläre Rate größer ist als beide VT-Zonen-Grenzwerte.

Die VT/SVT-Diskriminierungseinheit ist außerdem vorzugsweise dazu ausgebildet, einen Vergleich von für atriale Herzaktionen charakteristischen Signalmerkmalen durchzuführen, die einerseits bei normalem Rhythmus aufgezeichnet wurden und andererseits während einer Tachykardie aufgezeichnet wurden.

Weitere bevorzugte Ausführungsvarianten ergeben sich durch Kombination der hier beschriebenen Merkmale sowie der in der nachfolgenden Beschreibung eines Ausführungsbeispiels genannten Merkmale.

Die Erfindung soll nun anhand eines Ausführungsbeispiels mit Bezug auf die Figuren näher erläutert werden. Von diesen zeigt:
- Fig.1:: einen Herzstimulator in Form eines implantierbaren Einkammer-Kardioverter/Defibrillator in Verbindung mit daran angeschlossenen Elektrodenleitungen;
- Fig. 2:: ein schematisches Blockschaltbild des Herzstimulators aus Figur 1;
- Fig. 3:: die für die VT/SVT Diskriminierung vorgesehenen Komponenten des Herzstimulators aus Figuren 1 und 2 in detaillierter Darstellung;
- Fig. 4:: die Ergebnisse einer Signalmittelung (Averaging) zur Erkennung einer P-Welle im Far-Field-Elektrogramm;
- Fig. 5:: eine Aufzeichnung einer Referenz-P-Welle und einer Tachykardie-P-Welle; und
- Fig. 6:: ein Flussdiagram eines möglichen VT/SVT-Diskriminierungsalgorithmus.

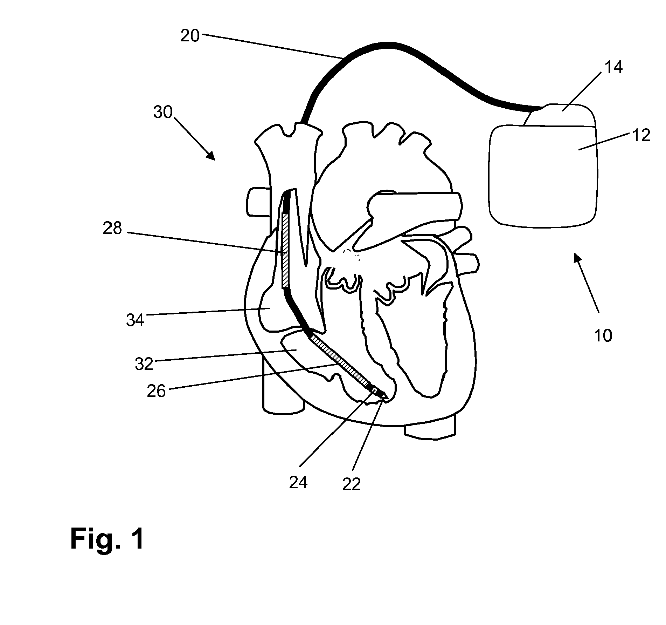

Figur 1 zeigt einen ventrikulären Herzstimulator 10 mit einem Gehäuse 12 und einem Header 14. Das Gehäuse 12 ist hohl und besitzt wenigstens teilweise eine elektrisch leitende Oberfläche, typischer Weise besteht das Gehäuse 12 aus einem biokompatiblen Metall wie Titan. In dem Gehäuse 12 befinden sich eine Batterie und elektronische Komponenten des Herzstimulators 10. Der Header 14 enthält Anschlussbuchsen, beispielsweise für eine Elektrodenleitung.

In Figur 1 ist der Herzstimulator 10 mit einer flexiblen, implantierbaren Elektrodenleitung 20 verbunden.

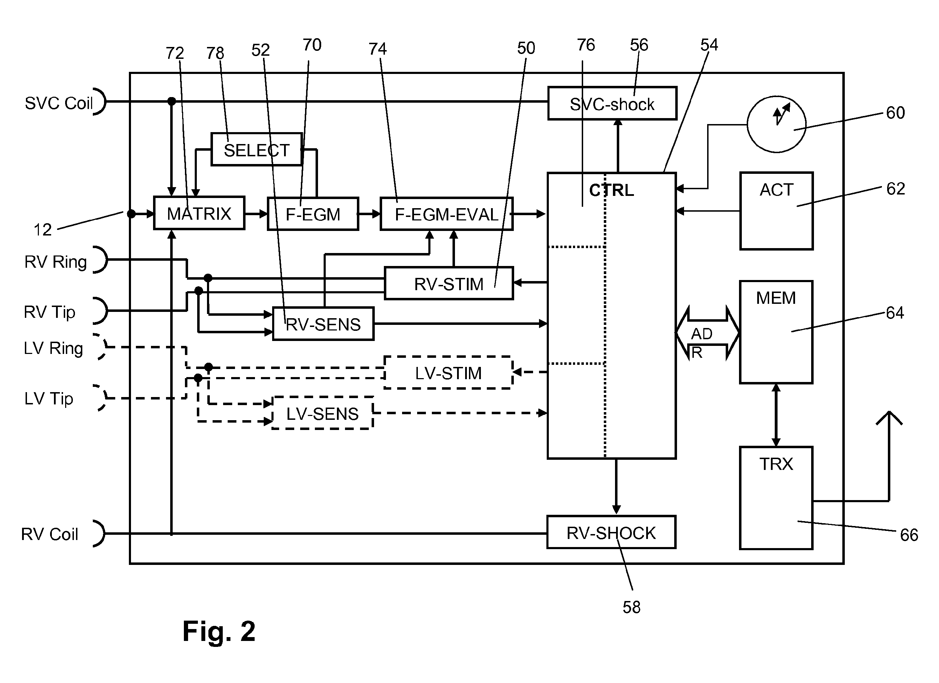

Die abgebildete Elektrodenleitung 20 ist eine ventrikuläre Elektrodenleitung, die an ihrem distalen Ende eine ventrikuläre Tip-Elektrode 22 und eine ventrikulären Ringelektrode 24 aufweist, die zusammen einen bipolare Wahrnehmungs- und Stimulationspol bilden. Von diesen dient die ventrikuläre Tip-Elektrode 22 als Stimulationselektrode. Die ventrikuläre Tip-Elektrode 22 und die ventrikuläre Ringelektrode 24 bilden zusammen ein Elektrodenpaar für das bipolare Sensing ventrikulärer Ereignisse. Hierzu sind die ventrikuläre Tip-Elektrode 22 und die ventrikuläre Ringelektrode 24 mit einer ventrikulären Stimulationseinheit und einer ventrikulären Sensingeinheit im Inneren des Gehäuses 12 des Herzstimulators 10 verbunden (Näheres ist mit Bezug auf Fig. 2 beschrieben). Außerdem besitzt die ventrikuläre Elektrodenleitung 20 eine ventrikuläre Schockwendel 26 als distale Schockwendel und eine proximale Schockwendel 28. Die ventrikuläre Schockwendel 26 ist dabei so auf der ventrikulären Elektrodenleitung 20 angeordnet, dass sie dann, wenn sie in ein Herz 30 eingeführt ist, im Ventrikel 32 des Herzens angeordnet ist. Die ventrikuläre Tip-Elektrode 22 befindet sich dann im Apex des Ventrikels 32 des Herzens 30. Die proximale Schockwendel 28 befindet sich bei implantierter ventrikulärer Elektrodenleitung 20 in der Vena cava superior des Herzens 30. Eine spezielle Elektrodenleitung zur Stimulation des rechten Atriums 34 des Herzens 30 ist nicht vorgesehen. Ebenso gibt es keine atriale Sensingelektrode. Der Herzstimulator 10 kommt ganz ohne atriale Elektrodenleitung aus und bietet dennoch die Funktionalität solcher Herzstimulatoren, die mit einer atrialen Elektrodenleitung verbunden sind. Dies wird anschließend näher erläutert.

Figur 2 zeigt in einem schematischen Blockschaltbild Komponenten des Herzstimulators 10, die im Inneren des Gehäuses 12 angeordnet sind. Dabei ist die Darstellung nicht notwendiger Weise abschließend. Insbesondere sind in Fig. 2 Komponenten gestrichelt dargestellt, die bei einem Herzstimulator 10 aus Fig. 1 nicht verwirklicht sind.

Wie bereits angedeutet besitzt der Herzstimulator 10 in seinem Header 14 Anschlusskontakte für den Anschluss entsprechender Gegenkontakte eines Elektrodenleitungssteckers am proximalen Ende der Elektrodenleitung 20. Diese Kontakte dienen zur elektrischen Verbindung mit den Elektroden der Elektrodenleitung 20. So ist die proximale Schockwendel 38 mit dem Kontakt SVC Coil verbunden, die ventrikuläre (distale) Schockwendel 26 mit dem Anschluss RV Coil, die rechtsventrikuläre Tip-Elektrode 22 mit dem Anschluss RV Tip und die rechtsventrikuläre Ringelektrode mit dem Anschluss RV Ring. Über die Anschlüsse RV Tip und RV Ring sind die rechtsventrikuläre Tip-Elektrode 22 und die rechtsventrikuläre Ringelektrode 24 jeweils mit einer rechtsventrikulären Stimulationseinheit 50 und einer rechtsventrikulären Sensingeinheit 52 verbunden. Die rechtsventrikuläre Sensingeinheit 52 ist dabei ausgangsseitig mit einer Stimulationssteuereinheit 54 verbunden, die ihrerseits wiederum einen Ausgang besitzt, der mit der rechtsventrikulären Stimulationseinheit 50 verbunden ist. Die rechtsventrikuläre Stimulationseinheit 50 ist dazu ausgebildet, auf ein entsprechendes Steuersignal der Stimulationssteuereinheit 54 hin einen ventrikulären Stimulationsimpuls zu generieren und wenigstens über den Kontakt RV Tip abzugeben.

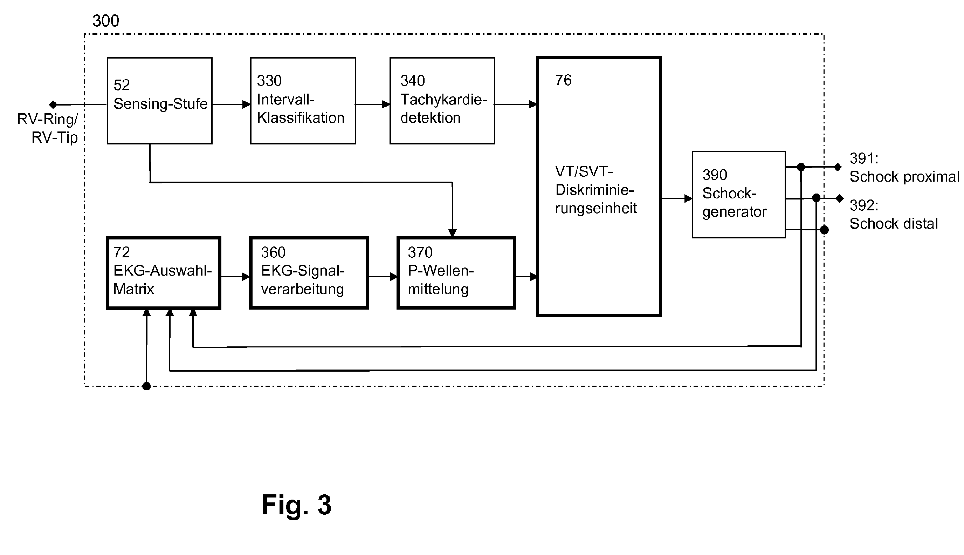

Die Funktionsweise der rechtsventrikulären Sensingeinheit 52 wird nachfolgend in Bezug auf Figur 3 noch näher erläutert. Die rechtsventrikuläre Sensingeinheit ist grundsätzlich dazu ausgebildet, den Verlauf eines Signals auszuwerten, der sich aus der Differenz der an den Anschlüssen RV Tip und RV Ring anliegenden Potenziale ergibt. Dieser Signalverlauf enthält typischer Weise Signalspitzen, die im Falle ventrikulärer Depolarisationen auftreten. Ventrikuläre Depolarisationen gehen einer Kontraktion des ventrikulären Myokards voraus und kennzeichnen somit ventrikuläre Senseereignisse. Diese können aus dem Signalverlauf detektiert werden, indem die Potenziale mit einem Schwellwert verglichen werden. Dieser ist so eingestellt, dass die mit ventrikulären Depolarisationen einhergehenden Signalspitzen den Schwellwert überschreiten, so dass die ventrikuläre Sensingeinheit 52 ventrikuläre Senseereignisse durch Schwellwertvergleich bei Schwellwertüberschreitung detektieren kann.

Zur Erzeugung und Abgabe von Defibrillationsschocks sind außerdem Schockgeneratoren 56 und 58 vorgesehen, die über den Anschluss SVC Coil mit der proximalen Schockwendel 28 bzw. über den Anschluss RV Coil mit der distalen Schockwendel 26 verbunden sind. Die beiden Defibrillationsschock-Generatoren 56 und 58 sind dabei jeweils ebenfalls mit der Stimulationssteuereinheit 54 verbunden. Weitere Merkmale des Herzstimulators 10 sind ein Zeitgeber 60, der beispielsweise zur Intervallmessung und Herzratenbestimmung herangezogen wird, sowie ein Aktivitätssensor 62, der ausgebildet ist, eine physische Aktivität eines Patienten, beispielsweise durch Bewegungserfassung, zu erfassen, um es der Stimulationssteuereinheit 54 zu ermöglichen, eine Stimulationsrate an den physiologischen Bedarf eines Patienten anzupassen. Weiterhin besitzt der Herzstimulator 10 einen Speicher 64 zum Speichern von Steuerparametern sowie von physiologischen Parametern, die beispielsweise durch Auswerten der verschiedenen vom Herzstimulator 10 aufgenommenen Signale gewonnen werden.

Schließlich besitzt der Herzstimulator 10 auch eine Telemetrieeinheit 66, über die der Herzstimulator 10 gewonnene und gespeicherte physiologische Parameter an ein externes Gerät drahtlos übermitteln kann oder aber über die der Herzstimulator 10 Steuerparameter empfangen kann, die die Funktionsweise des Herzstimulators 10 steuern.

Im Sinne der Erfindung besitzt der Herzstimulator 10 außerdem eine Fernfeldelektrokardiogramm-Erfassungseinheit 70, die eingangsseitig einerseits mit einer Schaltmatrix 72 verbunden ist, über die die Fernfeldelektrokardiogramm-Erfassungseinheit 70 wahlweise einerseits mit dem elektrisch leitenden Gehäuse 12 des Herzstimulators 10 und andrerseits über den Anschluss SVC Coil mit der proximalen Schockwendel 28 oder über den Anschluss RV Coil mit der distalen Schockwendel 26 oder über den Anschluss RV Ring mit der rechtsventrikuläre Ringelektrode 24 oder über den Anschluss RV Tip mit der rechtsventrikulären Tip-Elektrode 22 zu verbinden ist.

Grundsätzlich ergeben sich die folgenden Elektrodenkonfigurationen für das Eingangssignal der Fernfeldelektrokardiogramm-Erfassungseinheit 70:
- Variante A:: RV-Tip - Gehäuse
- Variante B:: RV-Ring - Gehäuse
- Variante C:: RV Coil - Gehäuse
- Variante D:: SVC Coil - Gehäuse

Die bevorzugten Ableitungen sind jedoch Variante D, sofern eine proximale Schockwendel vorhanden ist und Variante C, wenn keine proximale Schockwendel vorhanden ist, da in diesen Ableitungen die Abbildung der Vorhoferregung (P-Welle) am günstigsten ist.

Das mehrere Ableitungen für die Bestimmung der P-Welle möglich sind, ist in dem Ausführungsbeispiel die Schaltmatrix 72 vorgesehen, die entweder manuell durch den Anwender programmierbar, oder aber automatisch anhand von Elektrodenimpedanzen und Signalqualität die jeweils günstigste Ableitung für die P-Wellenbestimmung auswählt. Zum letztgenannten Zweck ist eine Auswahleinheit 78 vorgesehen, die die von der Fernfeldelektrokardiogramm-Erfassungseinheit 70 generierten Fernfeld-Elektrokardiogrammsignale bewertet und entsprechend die Schaltmatrix 72 ansteuert. Dazu ist die Auswahleinheit 78 eingangsseitig mit der Fernfeldelektrokardiogramm-Erfassungseinheit 70 und ausgangsseitig mit der Schaltmatrix 72 verbunden.

Aus der Potenzialdifferenz zwischen den beiden ausgewählten Eingängen ergibt sich ein Fernfeldelektrokardiogramm, das von der Fernfeldelektrokardiogramm-Erfassungseinheit 70 aufgenommen, verstärkt, analog-digital-gewandelt und gefiltert wird, wie dies mit Bezug auf Figur 3 noch näher erläutert wird. Das so gewonnene und aufbereitete Fernfeldelektrokardiogramm liegt an einem Ausgang der Fernfeldelektrokardiogramm-Erfassungseinheit 70 an. Dieser Ausgang ist mit einem Eingang einer Fernfeldelektrokardiogramm-Auswerteeinheit 74 verbunden. Diese Fernfeldelektrokardiogramm-Auswerteeinheit 74 besitzt außerdem noch einen Eingang, der mit der rechtsventrikulären Sensingeinheit 52 sowie einen weiteren Eingang, der mit der rechtsventrikulären Stimulationseinheit 50 verbunden ist. Alternativ dazu kann auch ein einziger Eingang der Fernfeldelektrokardiogramm-Auswerteeinheit 74 vorgesehen sein, der mit der Stimulationssteuereinheit 54 verbunden ist. Diese weiteren Eingänge der Fernfeldelektrokardiogramm-Auswerteeinheit 74 dienen dazu, der Fernfeldelektrokardiogramm-Auswerteeinheit 74 Signale zuzuführen, die ventrikuläre Sensingereignisse bzw. ventrikuläre Stimulationsereignisse charakterisieren. Die Fernfeldelektrokardiogramm-Auswerteeinheit 74 ist ausgebildet, das seitens der Fernfeldelektrokardiogramm-Erfassungseinheit 70 gebildete Fernfeldelektrokardiogramm unter Berücksichtigung ventrikulärer Sensingereignisse und Stimulationsereignisse charakterisierender Signale auszuwerten, um in dem Fernfeldelektrokardiogramm Signalmerkmale zu detektieren, die atriale (Sense-)Ereignisse charakterisieren. Solche Signalmerkmale werden als P-Wellen bezeichnet.

Die Fernfeldelektrokardiogramm-Auswerteeinheit 74 ist ausgangsseitig mit einer VT/SVT-Diskriminierungseinheit 76 als Teil der Stimulationssteuereinheit 54 verbunden.

In Figur 3 sind diejenigen Komponenten eines Einkammer-ICD dargestellt, die eine erfindungsgemäße VT/SVT-Diskriminierung bewirken.

Der ICD ist mit einer rechtsventrikulären Elektrodenleitung 20 als Wahrnehmungs- und Stimulationselektrode verbunden. Das mittels der Elektroden 22 und 24 dieser Elektrodenleitung 20 abgeleitete intrakardiale Elektrogramm wird in einer konventionellen ICD-Sensingstufe 52 analysiert und die ventrikulären Intervalle (RR-Intervall) werden vermessen. Diese RR-Intervalle werden anschließend in einer Intervallklassifikationseinheit 330 entsprechend ihrer Zykluslänge klassifiziert und mindestens einer Tachykardiezone zugeordnet. Fällt ein RR-Intervall in eine Tachykardiezone, wird in der Tachykardiedetektionseinheit 340 ein Zähler entsprechend hochgezählt. Wird ein vorgegebenes Zählerkriterium für die Tachykardiedetektion in einer Zone erfüllt, so wird die nachfolgende VT/SVT-Diskriminierungseinheit 76 aktiviert.

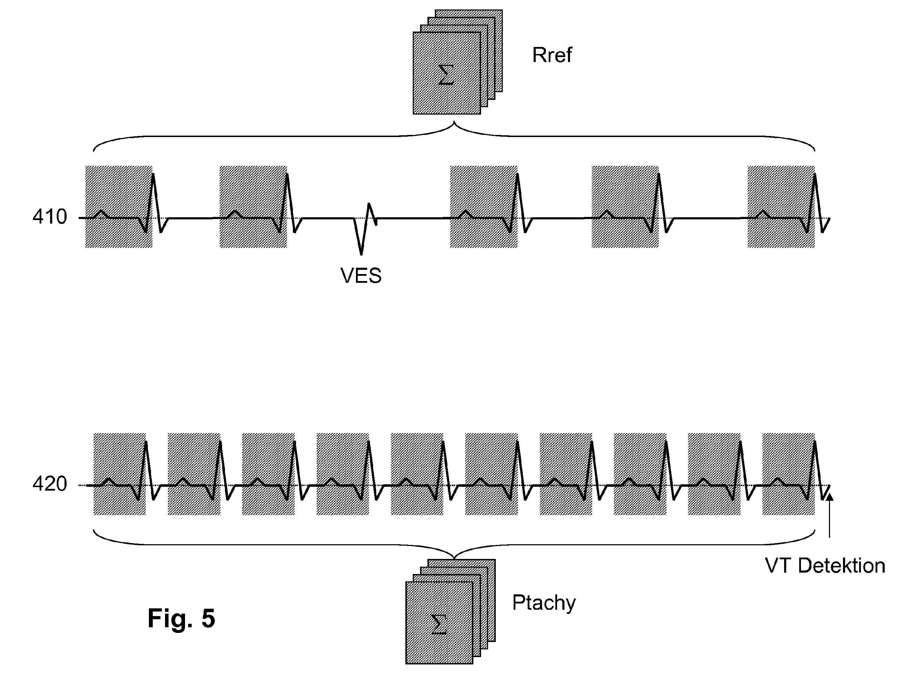

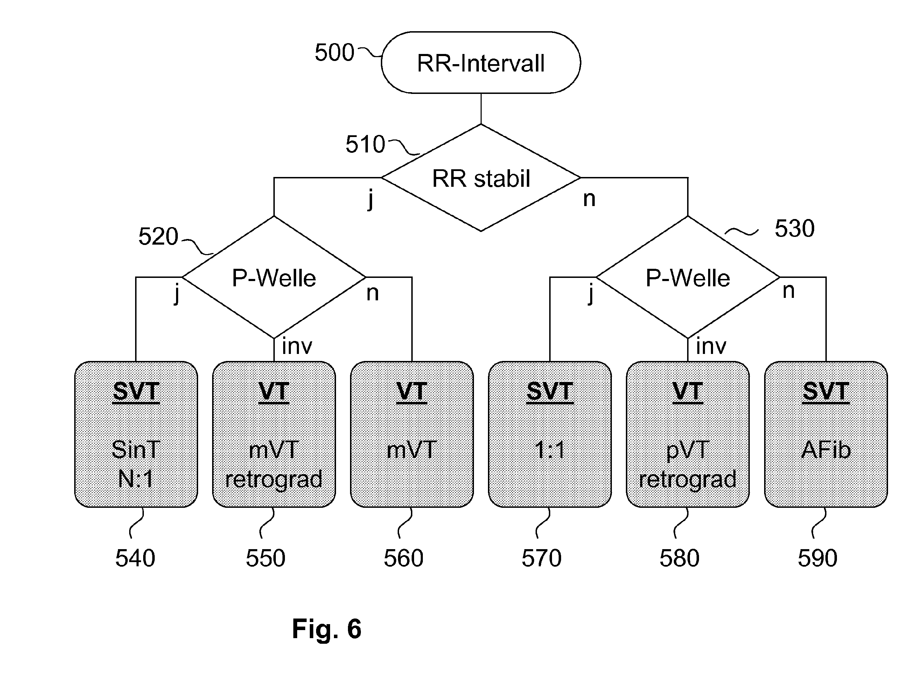

Die mit dem Schockgenerator 56/58 des ICD verbundenen Schockelektroden 26 und 28 und das elektrisch leitfähige Gehäuse 12 sind zusätzlich mit einer Far-Field-EKG-Auswahl-Schaltmatrix 72/78 verbunden. Diese Auswahl-Schaltmatrix 72/78 legt fest, welche der Elektroden für die Ableitung eines Far-Field-Elektrokardiogrammsignals zur VT/SVT-Diskriminierung verwendet werden. Die Auswahl erfolgt dabei entweder manuell durch eine Programmierung durch den Arzt oder aber automatisch durch eine im ICD durchgeführte Signalqualitätsanalyse ("Signal Quality Check"). ein so gewonnenes Far-Field-Elektrokardiogrammsignal wird anschließend in einer Elektrokardiogrammsignal-Verarbeitungseinheit 360 vorverarbeitet (verstärkt, digitalisiert, gefiltert) und dann einem Mittelwertbildner 370 zugeführt. Dieser Mittelwertbildner führt eine auf eine von der Sensingeinheit 52 detektierte ventrikuläre Herzaktion hin getriggerte Signalmittelung durch und analysiert den Elektrokardiogramm-Signalabschnitt vor der detektierten ventrikulären Herzaktion, um so eine P-Welle als für atriale Herzaktionen charakteristische Signalmerkmal zu erkennen und zu klassifizieren. Die so erkannte P-Welle - getrennt nach Referenz-P-Welle und P-Welle zum Zeitpunkt einer Tachykardiedetektion (Tachykardie-P-Welle) - wird der VT/SVT-Disktiminierungseinheit 76 zugeführt. In der VT/SVT-Diskriminierungseinheit 76 wird der Rhythmus entsprechend dem in Figur 6 gezeigten Entscheidungsbaum klassifiziert, wobei hier immer ein Vergleich zwischen der vor der Tachykardie aufgezeichneten P-Welle und der während der Tachykardie aufgezeichneten P-Welle (siehe Figur 5) stattfindet.

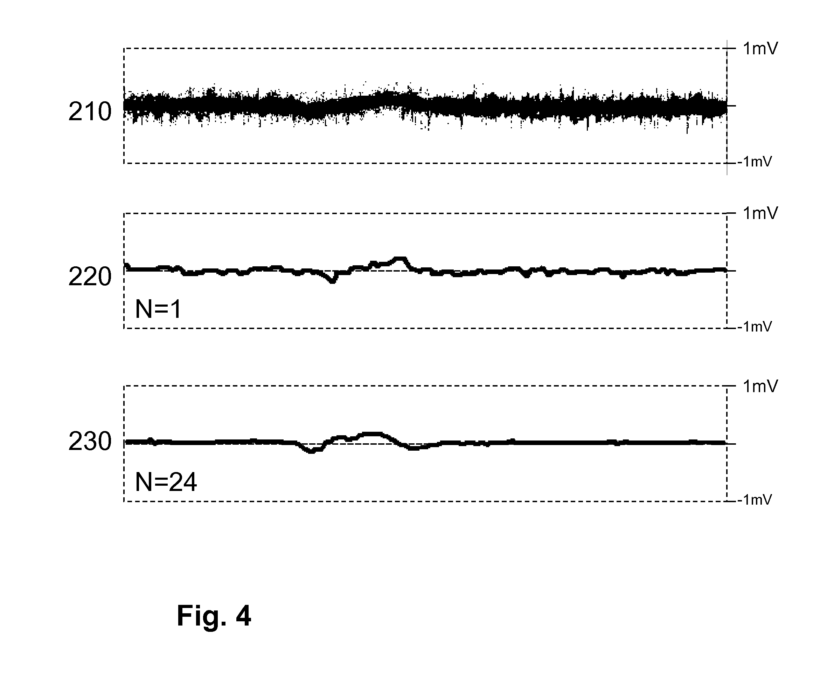

Figur 4 zeigt die Ergebnisse der Signalmittelung (Averaging) zur Erkennung einer P-Welle im Far-Field-Elektrogramm.

Die erste, obere Kurve (210) zeigt ein Eingangssignal mit einer Amplitude von 0,1 mV, um eine sehr kleine P-Welle zu simulieren. Zusätzlich ist das Eingangssignal etwas verrauscht, um so die realen Bedingungen der Far-Field-EKG-Ableitung zu simulieren.

In der zweiten, mittleren Kurve (220) wird die Ableitung dieser P-Wellen im Far-Field-Elektrogramm, abgeleitet mit einem ICD zwischen der proximalen Schockwendel und dem Gehäuse des ICD dargestellt. Die neben der zu erkennenden P-Welle abgebildeten Störsignale lassen keine automatische P-Wellenerkennung durch den ICD zu.

In der dritten, unteren Kurve (230) ist das Signal dargestellt nachdem es über 24 Herzzyklen gemittelt wurde. Diese Signalqualität ist hinreichend, um eine automatische Erkennung der P-Welle im ICD zu realisieren.

In Figur 5 ist die Aufzeichnung der Referenz-P-Welle und der Tachykardie-P-Welle dargestellt. Während eines Herzrhythmus mit einer Frequenz kleiner 100 ppm (410) erfolgt die Aufzeichnung der Referenz-P-Wellen als gleitende Mittelwert Bildung über eine festgelegte Anzahl von Intervallen (z.B. 24), um so immer ein aktuelles Referenztemplate einer P-Welle verfügbar zu haben. Extrasystolen (VES) oder stimulierte Ereignisse gehen dabei nicht in die Mittelwertbildung ein.

Wird eine Tachykardie festgestellt, erfolgt die Mittelwertbildung in einem separaten Mittelwertbildner für Intervalle innerhalb der Tachykardiezone, so dass zum Zeitpunkt einer erfüllten Tachykardiedetektion eine gemittelte P-Wellenaufzeichnung für die VT/SVT-Klassifikation verfügbar ist.

Kann keine P-Welle im Referenzsignal nachgewiesen werden, so wird anstelle des nachfolgend in Figur 6 dargestellten VT/SVT-Diskriminierungsalgorithmus eine "normale" Einkammer-Diskriminierung durchgeführt.

Figur 6 zeigt in Form eines Flussdiagramms einen möglichen VT/SVT-Diskriminierungsalgorithmus, der auf einer P-Wellenmittelung vor und während einer Tachykardie basiert.

Erreicht der Detektionszähler den für die Tachykardieerkennung programmierten Zählerwert, werden alle RR-Intervalle (500), die in die Tachykardiezone gefallen sind entsprechend des folgenden Ablaufplans bewertet:

Zunächst erfolgt die Prüfung, ob die tachykarden RR-Intervalle ein Intervallstabilitätskriterium (510) erfüllen. Diese Stabilitätsprüfung erfolgt mit den bereits bekannten Verfahren zur Intervallstabilitätsprüfung.

Wird die Tachykardie als stabil bewertet, erfolgt anschließend die Prüfung (520), ob im Vergleich zur Referenz-P-Welle (siehe Abbildung 4) die P-Welle, gemittelt während der Tachykardie entweder der Referenz-P-Welle entspricht oder inhibiert wird (Polarität getauscht) oder aber nicht mehr nachweisbar ist.

Entspricht die gemittelte Tachy-P-Welle der Referenz, dann wird die Tachykardie als SVT bewertet. Es handelt sich dann entweder um eine Sinustachykardie oder eine atriale Tachykardie mit einem festen Überleitungsverhältnis (N:1) in den Ventrikel (540).

Ist die gemittelte Tachy-P-Welle gegenüber der Referenz inhibiert, dann wird die Tachykardie als VT bewertet. Es handelt sich dann um eine monomorphe ventrikuläre Tachykardie mit einer retrograden Überleitung in das Atrium (550).

Ist keine Tachy-P-Welle nachweisbar, dann wird die Tachykardie als VT bewertet. Es handelt sich dann um eine monomorphe ventrikuläre Tachykardie ohne retrograde Leitung in das Atrium (560).

Wird die Tachykardie als instabil bewertet (510), erfolgt anschließend die Prüfung (530), ob im Vergleich zur Referenz-P-Welle (siehe Abbildung 4) die P-Welle, gemittelt während der Tachykardie entweder der Referenz-P-Welle entspricht oder inhibiert wird (Polarität getauscht) oder aber nicht mehr nachweisbar ist.

Entspricht die gemittelte Tachy-P-Welle der Referenz, dann wird die Tachykardie als SVT bewertet. Es handelt sich dann um eine atriale Tachykardie mit einer 1:1 Überleitung in den Ventrikel (570).

Ist die gemittelte Tachy-P-Welle gegenüber der Referenz inhibiert, dann wird die Tachykardie als VT bewertet. Es handelt sich dann um eine polymorphe ventrikuläre Tachykardie mit einer retrograden Überleitung in das Atrium (580).

Ist keine Tachy-P-Welle nachweisbar, dann wird die Tachykardie als SVT bewertet. Es handelt sich dann um ein atriales Flimmern mit tachykarder Überleitung (590). Alternativ kann in diesem Entscheidungspfad auch eine Klassifikation als VT stattfinden. In diesem Fall wäre von eine polymorphen VT auszugehen. Da ein tachykard übergeleitetes Afib in der Regel langsamer als eine pVT ist, kann zusätzlich ein Frequenzkriterium verwendet werden, um entweder eine SVT oder VT im Pfad 590 zu klassifizieren. Alternativ kann diese Entscheidung auch an die jeweilige VT-Zone gebunden sein: in eine langsamen VT-Zone 590=SVT, in einer schnellen 590=VT.

## Patentansprüche

1. Einkammer-Herzstimulator mit
- einem wenigstens zum Teil elektrisch leitenden Gehäuse (12),
- einer ventrikulären Sensingeinheit, die über eine ventrikuläre Elektrodenleitung mit wenigstens einem ventrikulären Sensing-Elektrodenpaar verbunden oder zu verbinden ist und die ausgebildet ist, ventrikuläre Herzaktionen vermittels eines über ein ventrikuläres Sensing-Elektrodenpaar bipolar aufgenommenen ersten Elektrokardiogrammsignals wahrzunehmen und zu klassifizieren (detektieren),
- einer weiteren Erfassungseinheit, die einerseits über die ventrikuläre Elektrodenleitung mit wenigstens einer Elektrode dieser ventrikulären Elektrodenleitung verbunden oder zu verbinden ist und andrerseits mit dem elektrisch leitenden Gehäuse (12) des Einkammer-Herzstimulators verbunden und die ausgebildet ist, in einem über diese Elektroden aufgenommenen zweiten Elektrokardiogrammsignal für atriale Herzaktionen (P-Wellen) charakteristische Signalmerkmale zu detektieren und
- einer VT/SVT-Diskriminierungseinheit, die mit der ventrikulären Sensingeinheit und der weiteren Erfassungseinheit verbunden und ausgebildet ist, anhand von über die ventrikuläre Sensingeinheit erfassten ventrikulären Herzaktionen sowie von über die weitere Erfassungseinheit erfassten atrialen Herzaktionen (P-Wellen) eine Unterscheidung zwischen ventrikulären und supraventrikulären Tachykardien durchzuführen, wenn die Rate der erfassten ventrikulären Herzaktionen einen Grenzwert überschreitet.

2. Einkammer-Herzstimulator nach Anspruch 1, **dadurch gekennzeichnet, dass** die weitere Erfassungseinheit ausgebildet ist, für atriale Herzaktionen (P-Wellen) charakteristische Signalmerkmale vor und während einer Tachykardie zu detektieren.

3. Einkammer-Herzstimulator nach Anspruch 1 oder 2, **dadurch gekennzeichnet, dass** die weitere Erfassungseinheit ausgebildet ist für atriale Herzaktionen (P-Wellen) charakteristische Signalmerkmale durch Signalmittelung über mehrere Herzzyklen zu bestimmen.

4. Einkammer-Herzstimulator nach einem der Ansprüche 1 bis 3, **dadurch gekennzeichnet, dass** die weitere Erfassungseinheit mit einer Schockwendel an einer ventrikulären Elektrodenleitung als Elektrode dieser ventrikulären Elektrodenleitung verbunden oder zu verbinden ist.

5. Einkammer-Herzstimulator nach Anspruch 4, **dadurch gekennzeichnet, dass** die weitere Erfassungseinheit mit einer distalen, zur Platzierung im Ventrikel vorgesehenen Schockwendel an einer ventrikulären Elektrodenleitung als Elektrode dieser ventrikulären Elektrodenleitung verbunden oder zu verbinden ist.

6. Einkammer-Herzstimulator nach Anspruch 4, **dadurch gekennzeichnet, dass** die weitere Erfassungseinheit mit einer proximalen, zur Platzierung in der Vena Cava Superior vorgesehenen Schockwendel an einer ventrikulären Elektrodenleitung als Elektrode dieser ventrikulären Elektrodenleitung verbunden oder zu verbinden ist.

7. Einkammer-Herzstimulator nach einem der Ansprüche 1 bis 3, **dadurch gekennzeichnet, dass** die weitere Erfassungseinheit mit einer ventrikulären Spitzenelektrode an einer ventrikulären Elektrodenleitung als Elektrode dieser ventrikulären Elektrodenleitung verbunden oder zu verbinden ist.

8. Einkammer-Herzstimulator nach einem der Ansprüche 1 bis 3, **dadurch gekennzeichnet, dass** die weitere Erfassungseinheit mit einer ventrikulären Ringelektrode an einer ventrikulären Elektrodenleitung als Elektrode dieser ventrikulären Elektrodenleitung verbunden oder zu verbinden ist.

9. Einkammer-Herzstimulator nach einem der Ansprüche 1 bis 8, **dadurch gekennzeichnet, dass** die weitere Erfassungseinheit über eine Schaltmatrix wahlweise mit wenigstens einer von mehreren Elektroden einer ventrikulären Elektrodenleitung verbunden oder zu verbinden ist.

10. Einkammer-Herzstimulator nach Anspruch 9, **dadurch gekennzeichnet, dass** Schaltmatrix mit einer Auswahleinheit verbunden ist, die ausgebildet ist, die Schaltmatrix basierend auf einer EKG-Signalqualitätsprüfung automatisch so umzuschalten, dass die weitere Erfassungseinheit automatisch mit derjenigen Elektrode der ventrikulären Elektrodenleitung verbunden wird, die das geeignetste Elektrokardiogrammsignal liefert.

11. Einkammer-Herzstimulator nach einem der Ansprüche 1 bis 10, **dadurch gekennzeichnet, dass** die weitere Erfassungseinheit dazu ausgebildet ist, mehrere zweite Elektrokardiogrammsignale gleichzeitig aufzunehmen und miteinander zu mitteln.

12. Einkammer-Herzstimulator nach einem der Ansprüche 1 bis 11, **dadurch gekennzeichnet,**
- **dass** der Herzstimulator eine Zeitmesseinheit aufweist, die ausgebildet ist, den zeitlichen Abstand aufeinander folgender ventrikulärer Herzaktionen als jeweiliges RR-Intervall oder dessen Kehrwert als jeweilige ventrikuläre Rate zu bestimmen und den so bestimmten Wert der VT/SVT-Diskriminierungseinheit (76) zuzuführen, und
- **dass** die VT/SVT-Diskriminierungseinheit (76) ausgebildet ist, ein jeweiliges RR-Intervall oder eine jeweilige ventrikuläre Rate mit wenigstens einem VT-Zonen-Grenzwert zu vergleichen und für den Fall, dass ein jeweiliges RR-Intervall kürzer ist, als der entsprechende VT-Zonen-Grenzwert oder eine jeweilige ventrikuläre Rate größer ist, als der entsprechende VT-Zonen-Grenzwert, eine Tachykardie zu detektieren und in dem Fall dass ein jeweiliges RR-Intervall länger ist, als der entsprechende VT-Zonen-Grenzwert oder eine jeweilige ventrikuläre Rate geringer ist, als der entsprechende VT-Zonen-Grenzwert, einen Normalrythmus zu detektieren.

13. Herzstimulator nach Anspruch 12, **dadurch gekennzeichnet, dass** die VT/SVT-Diskriminierungseinheit ausgebildet ist, ein jeweiliges RR-Intervall oder eine jeweilige ventrikuläre Rate mit wenigstens zwei VT-Zonen-Grenzwerten zu vergleichen und eine Tachykardie zu detektieren, wenn das jeweilige RR-Intervall kürzer ist, als ein erster VT-Zonengrenzwert und länger als ein zweiter VT-Zonen-Grenzwert bzw. die jeweilige ventrikuläre Rate größer als ein erster VT-Zonengrenzwert und kleiner als ein zweiter VT-Zonen-Grenzwert ist, und das jeweilige RR-Intervall oder eine ventrikuläre Fibrillation zu detektieren, wenn das jeweilige RR-Intervall kürzer ist, als der erste und der zweite VT-Zonen-Grenzwert bzw. die jeweilige ventrikuläre Rate größer als der erste und der zweite VT-Zonen-Grenzwert ist.

14. Herzstimulator nach Anspruch 12 oder 13, **dadurch gekennzeichnet, dass** die VT/SVT-Diskriminierungseinheit ausgebildet ist einen Vergleich von für atriale Herzaktionen charakteristischen Signalmerkmalen, die bei normalem Rhythmus aufgezeichnet sind mit solchen für atriale Herzaktionen charakteristischen Signalmerkmalen durchzuführen, die während einer Tachykardie aufgezeichnet wurden.
